# FASCICULE DE BREVET EUROPEEN

(11) **EP 3 429 587 B1**
(45) Date de publication et mention de la délivrance du brevet: **19.08.2020**
(21) Numéro de dépôt: 17710577.2
(22) Date de dépôt: 17.03.2017
(51) Int. Cl.: A61K 31/4741, A61K 9/20, A61K 9/28, A61K 9/48, A61P 11/00

(54) **UTILISATION D'UNE MOLÉCULE H4 AGONISTE POUR LE TRAITEMENT DE LA FIBROSE PULMONAIRE IDIOPATHIQUE**
VERWENDUNG EINES H4-AGONISTENMOLEKÜLS ZUR BEHANDLUNG VON IDIOPATHISCHER LUNGENFIBROSE
USE OF AN H4 AGONIST MOLECULE FOR THE TREATMENT OF IDIOPATHIC PULMONARY FIBROSIS

(30) Priorité: 18.03.2016 FR 1652332; 18.03.2016 US 201662310033 P
(43) Date de publication de la demande: 23.01.2019
(73) Titulaire: H4 Orphan Pharma, 21000 Dijon (FR)
(72) Inventeur: TERRASSE, Gaëtan, 71230 Saint Vallier (FR); BUR, Catherine, 78910 Orgerus (FR)
(74) Mandataire: Oudin, Stéphane
(86) Numéro de dépôt international: PCT/EP2017/056434
(87) Numéro de publication internationale: WO 2017/158179

(56) Documents cités:
- EP-A1- 2 659 890
- WO-A1-2006/100392
- WO-A1-2009/155611
- WO-A2-2006/131737
- RICHARD H. GOMER: "New Approaches to Modulating Idiopathic Pulmonary Fibrosis", CURRENT ALLERGY AND ASTHMA REPORTS, vol. 13, no. 6, 21 août 2013 (2013-08-21), pages 607-612, XP055312083, US ISSN: 1529-7322, DOI: 10.1007/s11882-013-0377-5

## Description

La présente invention se rapporte à l'utilisation de substances chimiques les énantiomères lévogyres et dextrogyres de la (AMINO-7 TRIETHOXY-4,5,6 OXO-1 DIHYDRO-1,3 ISOBENZOFURANNYL-3)-1 METHOXY-8 METHYL-2METHYLENEDIOXY-6,7 TETRAHYDRO-,2,3,4 ISOQUINOLEINE ou tritoqualine pour traiter la fibrose pulmonaire idiopathique.

La fibrose pulmonaire idiopathique (FPI) est l'une des 200 maladies pulmonaires de la famille des pneumopathies interstitielles, qui touchent l'interstitium pulmonaire, le tissu situé entre les espaces alvéolaires des poumons.

La fibrose pulmonaire idiopathique est une pneumopathie interstitielle idiopathique, qui est à son tour une pneumopathie interstitielle, aussi connue comme pneumopathie parenchymateuse diffuse. La fibrose pulmonaire idiopathique est une maladie rare (prévalence de l'ordre 13 à 20 cas/100 000 habitants) qui appartient au groupe des pneumopathies interstitielles diffuses idiopathiques d'évolution chronique, dont elle est la forme la plus fréquente (60% des cas) et la plus sévère.

Les pneumopathies interstitielles diffuses idiopathiques surviennent en l'absence de cause ou de contexte spécifique identifié, par opposition aux pneumopathies interstitielles diffuses dont la cause médicale est connue (pneumopathies interstitielles d'origine médicamenteuse ou pneumoconioses p.ex.).

La fibrose pulmonaire idiopathique a récemment été définie dans le cadre d'une classification de consensus international. Le terme de fibrose pulmonaire idiopathique recouvrait auparavant plusieurs entités qui sont maintenant bien distinctes, et en particulier la pneumopathie interstitielle non spécifique qui a été reconnue en tant qu'entité anatomoclinique au sein du groupe des pneumopathies interstitielles diffuses idiopathiques.

La définition de la fibrose pulmonaire idiopathique est maintenant restreinte aux cas de pneumopathies interstitielles diffuses idiopathiques où la biopsie pulmonaire montre un aspect histopathologique de pneumopathie interstitielle commune *(usuel interstitiel pneumonia).*

Des anomalies tomodensitométriques caractéristiques et un retentissement fonctionnel respiratoire sont également nécessaires pour retenir le diagnostic de fibrose pulmonaire idiopathique. Le diagnostic se fonde sur une synthèse anatomo-radio-clinique.

La fibrose pulmonaire idiopathique débute entre 60 et 70 ans avec une légère prédominance masculine. Elle est rare avant l'âge de 50 ans.

La maladie évolue progressivement vers l'insuffisance respiratoire chronique et le décès, avec une médiane de survie d'environ 3 ans, et une survie à 10 ans de l'ordre de 10% seulement.

La maladie est révélée par une dyspnée d'effort d'installation progressive, une toux non productive, et plus rarement des signes généraux. À l'examen, les râles crépitants secs bilatéraux des bases (reproduisant le bruit du «velcro») sont constants.

L'hippocratisme digital est présent dans près de la moitié des cas. La cyanose et les signes d'insuffisance ventriculaire droite ne s'observent qu'à un stade avancé de la maladie.

Le diagnostic est souvent porté tardivement, à un stade où les anomalies fonctionnelles et tomodensitométriques sont déjà marquées. La radiographie thoracique montre des opacités réticulaires périphériques prédominant nettement dans les bases, et une réduction du volume pulmonaire.

La tomodensitométrie thoracique en coupes millimétriques est l'examen essentiel du diagnostic : l'aspect caractéristique montre des opacités réticulaires des bases réalisant un aspect pseudo kystique sous-pleural en rayons de miel, des bronchectasies de traction, et des signes de distorsion du parenchyme pulmonaire, avec peu d'opacités en verre dépoli; L'exploration fonctionnelle respiratoire montre un trouble ventilatoire restrictif (diminution de la capacité vitale et de la capacité pulmonaire totale). Il existe en outre un trouble de fonctionnement de la membrane alvéolo-capillaire objectivée par une diminution du facteur et du coefficient de transfert du monoxyde de carbone (CO). L'hypoxémie est souvent absente au repos, mais elle apparaît à l'exercice; l'hypoxémie d'exercice est parfois la seule anomalie fonctionnelle respiratoire initialement.

Les patients ayant une fibrose pulmonaire idiopathique ont également un risque accru de cancer bronchique.

Si le pronostic global de la maladie est mauvais, il existe des différences interindividuelles importantes. Plusieurs études récentes ont permis d'identifier des facteurs pronostiques :
- La dégradation fonctionnelle respiratoire au cours des mois suivant le diagnostic est un facteur péjoratif. Ainsi, une diminution de la capacité vitale forcée de 10% ou plus en 6 mois, ou de la capacité de diffusion du CO de 15% ou plus en 12 mois, est un facteur prédictif indépendant d'un risque élevé de décès.
- L'existence d'une saturation inférieure à 88% lors d'un test de marche de 6 minutes est un facteur prédictif de mauvais pronostic.
- La survenue d'une hypertension pulmonaire au cours de l'évolution est aussi de mauvais pronostic.
- L'altération importante du facteur de transfert du CO.
- L'étendue des lésions en rayons de miel à la tomodensitométrie thoracique, et le nombre de foyers fibroblastiques sur la biopsie pulmonaire.

Le traitement de la fibrose pulmonaire idiopathique vise à améliorer l'espérance de vie, la fonction respiratoire, la qualité de vie, et à prévenir le risque d'exacerbation aiguë de la maladie.

Aucun traitement n'a, à ce jour, démontré son efficacité pour améliorer la survie dans cette affection. Il n'y a pas de traitement curatif à ce jour. Chez les patients s'aggravant et atteignant des stades sévères, l'oxygénothérapie et la transplantation pulmonaire sont les traitements recommandés, selon la Haute Autorité de Santé (HAS).

Deux médicaments ont cependant obtenus une Autorisation de Mise sur le Marché :
- D'une part, la pirfénidone (Esbriet) dont les résultats sur la survie sont peu ou non significatifs. Le mode d'action de la pirfénidone est inconnu. La pirfénidone est indiquée dans le traitement de la FPI légère à modérée. Ce produit est utilisé à des doses très élevées autour de 2,4 grammes/jour.
   Selon la HAS (Haute Autorité de Santé, l'efficacité de la pirfénidone a été appréciée selon un critère intermédiaire évaluant la fonction pulmonaire et marqueur de la progression de la maladie. La différence observée sur ce critère est en faveur de la pirfénidone par rapport au placebo mais cette différence est faible, de signification clinique mal connue et hétérogène d'une étude à l'autre. L'effet observé avec ESBRIET sur le déclin de la fonction pulmonaire chez les patients avec des critères fonctionnels précis (CVF ≥ 50% et DLCo ≥ 35%) est faible. Le bénéfice clinique apporté aux patients atteints de fibrose pulmonaire idiopathique est difficile à apprécier car les critères cliniquement pertinents (qualité de vie, survie globale...) ont fait l'objet d'analyses exploratoires et non robustes Cependant, des études de regroupement des patients ont permis de mettre en évidence sur certains profils patients une amélioration notable de la survie.
   Les principaux effets indésirables de la pirfénidone observés ont été des troubles gastro-intestinaux (nausée, diarrhée, dyspepsie), des troubles cutanés (photosensibilisation et rash) et des troubles du métabolisme et de la nutrition (anorexie et perte d'appétit).
- D'autre part, le nintedanib connu sous le nom commercial d'OFEV a été récemment approuvé aux USA pour le traitement de la fibrose pulmonaire idiopathique. Il a également reçu un avis favorable du CHMP de l'Agence Européenne du Médicament (EMA) et est en attente de l'Autorisation de Mise sur le Marché au niveau européen. Le mode d'action du nintedanib est celui d'un inhibiteur de tyrosine kinase. Ce produit est utilisé à la dose de 200 à 400 mg/jour. De nombreux effets secondaires ont été observés, notamment gastro-intestinaux.

La Haute Autorité de Santé considère que ne sont pas recommandés (absence d'étude de méthodologie acceptable, absence de démonstration sur des critères de jugement fonctionnels): corticoïdes en monothérapie, colchicine, ciclosporine A, interféron γ1b, bosentan, étanercept, association corticoïde/immunomodulateur et la trithérapie acétylcystéine/prednisone/azathioprine.

Un déséquilibre oxydant-antioxydant a été mis en évidence au cours de la fibrose pulmonaire idiopathique. La N-acétylcystéine (précurseur du glutathion, l'un des principaux antioxydants dans le poumon), a été évaluée comme traitement de la fibrose pulmonaire idiopathique.

Un essai randomisé contrôlé par la N-acétylcystéine (1800mg/j pendant un an) [en plus d'un traitement par corticoïdes et azathioprine] a récemment montré un bénéfice fonctionnel respiratoire modeste avec une diminution moyenne du déclin de la capacité vitale forcée de 0,18L (0,03-0,32) soit une différence relative de 9%, et de la diffusion du CO de 24% mais il n'existait pas de différence de mortalité entre les deux groupes.

La Haute Autorité de Santé considère que l'acétylcystéine, ayant montré un ralentissement de la détérioration de la capacité vitale avec un faible niveau de preuve, peut être administré à certains patients. Ce produit ne dispose pas d'une Autorisation de mise sur le Marché à ce jour.

De nombreux produits sont en cours d'étude pour le traitement de la fibrose pulmonaire idiopathique :
Des anticorps monoclonaux (lebrikizumab en phase II et tralokinumab en phase III) dont le statut en 2015 est incertain.

BMS-986202 (Lysophosphatidic Acid Receptor Antagonist) - développé par Bristol-Myers-Squibb est actuellement en phase II.

Plus de 154 études sont en cours mais peu de produits passent actuellement la phase II, car les résultats sont particulièrement médiocres.

Ainsi la fibrose pulmonaire idiopathique est actuellement une pathologie ayant peu de traitements disponibles commercialisés vraiment efficaces (uniquement ESBRIET à ce jour dans les FPI légères à modérées).

La tritoqualine est une substance chimique connue depuis de très nombreuses années, et utilisée comme antihistaminique. Sa fabrication est décrite dans le brevet français FR 1.295.309.

La tritoqualine est la 7-Amino-4,5,6-triethoxy-3-(5,6,7,8-tetrahydro-4-methoxy-6-methyl-1,3-dioxolo[4,5-g]isoquinolin-5-yl) phthalide. Dans sa forme pharmaceutique commercialisée, elle se présente sous la forme d'un mélange d'énantiomères.

La tritoqualine est connue pour son activité antiallergique par son action inhibitrice sur l'histidine décarboxylase. Cette activité est cependant très faible et n'explique pas les nombreuses propriétés qu'elle présente sur des symptômes cliniques variés, rhinite, urticaire, eczéma, mastocytose.

Un des inventeurs avec d'autres inventeurs a démontré que la tritoqualine avait une action très importante sur un nouveau récepteur, le récepteur H4 à l'histamine.

Cette activité de la tritoqualine sur le récepteur H4 a été démontrée dans un brevet US 2010144718A « TREATMENT OF DISEASES MODULATED BY A H4 RECEPTOR AGONIST » Gaëtan terrasse et coll.

Ce brevet ne décrit cependant pas l'activité de la tritoqualine sur la Fibrose pulmonaire idiopathique.

Un autre brevet WO2008006974A2 sur les agonistes H4 décrit l'utilisation de ces produits dans la protection des précurseurs hématopoïétiques dans le cadre de chimiothérapie.

Ce dernier brevet et aucun autre brevet ou document scientifique ne mettent en évidence l'action des agonistes H4 à l'histamine sur la Fibrose pulmonaire idiopathique.

La tritoqualine commerciale se présente sous la forme d'une poudre blanche, très sensible à la lumière qui la dégrade en Cotarnine et acide phtalique.

La tritoqualine commerciale (appelée Hypostamine) est sous la forme de comprimé avec une concentration de 100 mg par comprimé.

La tritoqualine présente 2 carbones asymétriques, mais la forme commerciale est un mélange de 2 énantiomères (R-R et S-S).

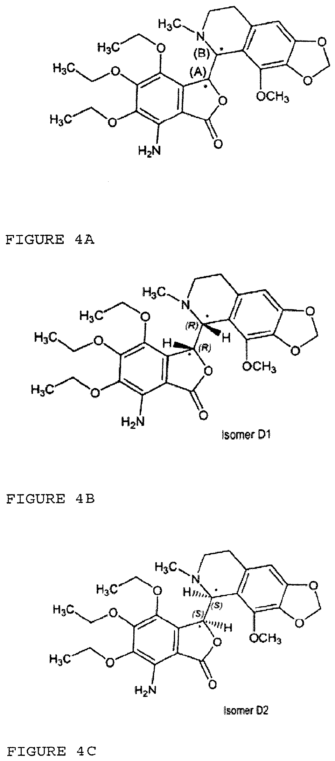

La Figure 4A illustre la présence des carbones asymétriques, qui sont notés A et B.

La Figure 4B illustre la forme de l'isomère D1.

La Figure 4C illustre La forme de l'isomère D2.

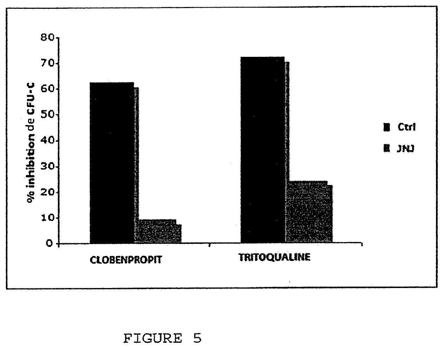

La figure 5 compare la tritoqualine et le Clobenpropit, agoniste H4 de référence. Cette figure met en évidence que la tritoqualine est capable d'inhiber la prolifération des CFU riche en récepteur H4. Quand on utilise un anti H4, l'activité de la tritoqualine est fortement diminuée comme celle du Clobenpropit. Ceci démontre bien que la tritoqualine est une molécule agoniste H4.

Les inventeurs ont mis en évidence les propriétés étonnantes et surprenante de la tritoqualine dans le modèle animal classique de la fibrose pulmonaire idiopathique, celle du modèle souris bléomycine.

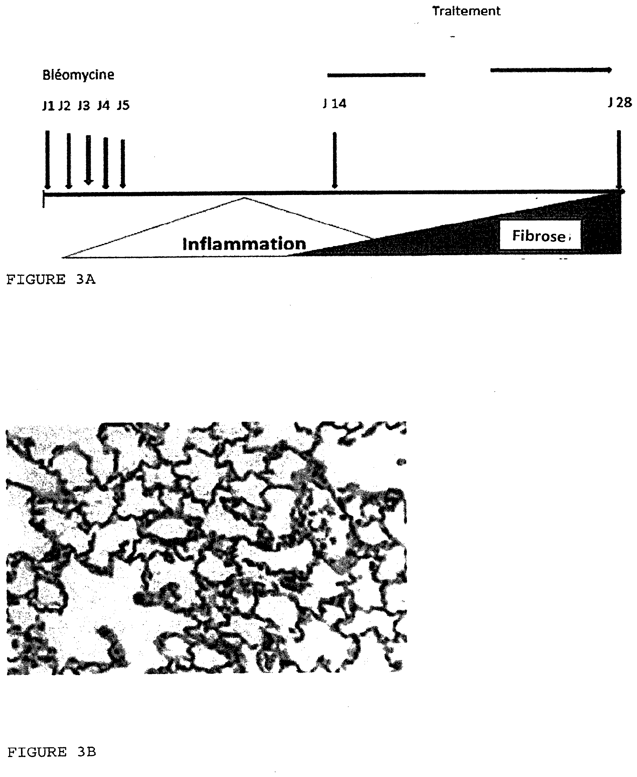

La figure 3 A explique le protocole utilisé. La bléomycine est inhalée pendant 5 jours pour provoquer une fibrose pulmonaire. A partir de J14 la tritoqualine est administrée per os tous les jours jusqu'à J28.

Les doses utilisées dans ce modèle sont de 20 et 40 mg/kg soit environ la dose chez un homme adulte de 70 kg de 1,4g/jour à 2,8 g/jour.

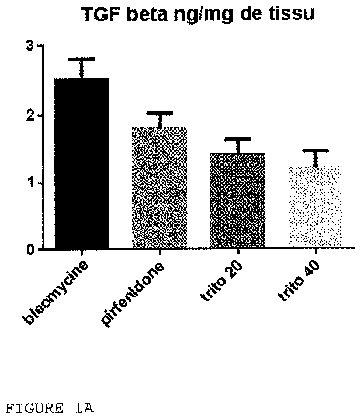

La figure 1A montre l'effet thérapeutique de la tritoqualine sur la sécrétion de TGF béta exprimée en ng/mg de tissu.

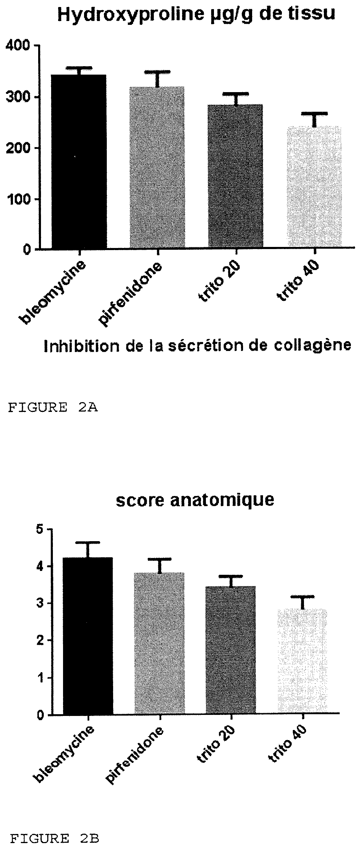

La figure 2A montre l'effet de la tritoqualine sur la sécrétion de collagène avec comme témoin la pirfenidone.
La figure 2B montre l'effet thérapeutique de la tritoqualine sur le score anatomique avec comme témoin la pirfenidone.
La figure 3B montre l'effet de la bléomycine sur le tissu pulmonaire.

### Exemple :

### Protocole

Le protocole bléomycine souris (BLM) a été effectué avec toutes les souris de chaque groupe de 10 souris (espèce : C57/Bl6J, dite plus loin comme BL6) ; il s'agit de souris femelles de 8 semaines d'âge.

L'analyse de la Fibrose a été effectuée avec tous les animaux de chaque groupe, respectivement. L'étendue de la fibrose a été analysée biochimiquement, histologiquement et cytologiquement en utilisant diverses méthodes quantitatives et semi quantitatives. Toutes les souris BL6 sont traitées selon le protocole ci-dessous.
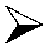 5 groupes de souris ont été évalués.

Le traitement par la bléomycine (BLM) se fait selon le protocole suivant :
Toutes les souris de tous les groupes (à l'exception du groupe 1) reçoivent de la bléomycine (BLM) par voie intranasale (3 mg /kg en instillation nasale) pendant 5 jours de suite et seront suivies quotidiennement et tuées à J + 29 après l'instillation BLM pour étudier l'inflammation pulmonaire chronique et la fibrose. La bléomycine étant administrée en cinq fois à l'âge de 8 semaines (voir schéma 3A).

Le traitement par la tritoqualine se fait à 2 doses, 20 et 40 mg/kg/jour pendant toute la durée de vie des souris. Le traitement de la tritoqualine commence quatorze jours après la première inhalation de traitement par la bléomycine. Le traitement se fait par voie orale. Les groupes étudiés sont les suivants :

| | |
|---|---|
| 1. BL6 mice | NaCl |
| 2. BL6 mice | BLM + vehicle |
| 3. BL6 mice | BLM + pirfenidone (100 mg/kg, po) |
| 4. BL6 mice | BLM + tritoqualine (20 mg/kg, po) |
| 5. BL6 mice | BLM + tritoqualine (40 mg/kg, po) |

### L'histologie

Après Lavage Broncho-Alvéolaire (LBA) et la perfusion du poumon, le grand lobe a été fixé dans du formaldéhyde tamponné à 4% pour une analyse microscopique standard (microscope Leica). Sections 3 mm ont été colorées avec du Trichome Masson (TM) en utilisant des techniques standards, qui permettent l'évaluation de la fibrose (TM) comme les fibres de collagène sont colorées en bleu et observer l'étendue de l'inflammation.

La fibrose a été évaluée par un score semi-quantitatif (0-5) (score Ashcroft) avec l'augmentation de la gravité par deux observateurs indépendants.

La fibrose avec le dépôt de collagène situé aux cloisons alvéolaires et dans le domaine péri bronchique et l'infiltration de cellules inflammatoires ont été évalués en utilisant un score semi-quantitatif de 0-5 avec une gravité croissante par deux observateurs indépendants.

### Titration du collagène (hydroxy proline)

Le taux de collagène se fait après homogénéisation du poumon. Après lavage Broncho alvéolaire (LBA), tout le poumon a été perfusé avec une solution saline à travers le ventricule cardiaque droit afin de vider le contenu vasculaire.

Les poumons ont été congelés à -70°C jusqu'à leur utilisation. Le poumon a ensuite été homogénéisé dans du PBS et centrifugé.

Le surnageant a été jeté et le culot a été remis en suspension dans 1 ml de PBS contenant 0,5% de bromure d'hexadécyltriméthylammonium (HTAB) et 5 mM d'éthylène-diamine tétra -acétique (EDTA).

Après la centrifugation, la teneur en collagène a été déterminée par le dosage Sircol (France Biochem Division, France), comme décrit par le fabricant.

### La mesure des cytokines

Les niveaux d'interféron gamma et de TGFβ ont été mesurés dans un homogénats de poumon ; les concentrations de ces différentes cytokines ont été déterminées par un dosage Luminex utilisant un MAGPIX (Biorad, France) selon les instructions du fabricant.

### Les résultats

La mesure histologique selon la méthode évaluée par un score semi-quantitatif (0-5) (score Ashcroft) donne les résultats suivants :

### Test pirfenidone 100 mg/kg versus bléomycine pour l'histologie.

- Paired t test (test de student)
- P value 0, 0449
- P value résumé: Le P < 0.05 Le test est-il significatif? Oui

Le test est-il hautement significatif? Non, la différence est faible (P= 0.045) à la limite de la significativité.

Moyenne ± écart type (bléomycine) : 4.2 ± 0.14, n=10
Moyenne ± écart type (Pirfenidone) : 3.8 ± 0,12 n=10

Différence entre les deux moyennes : -0,40 ± 0,18
Conclusion : efficacité de la pirfénidone à la limite de la significativité.

### Test tritoqualine 20 mg/kg versus bléomycine pour l'histologie.

- Paired t test (test de student)
- P value < 0, 0002
- P value résumé : Le P < 0.0002 Le test est-il significatif? Oui

Le test est-il hautement significatif? Oui, la différence est très élevée (P= 0.0002) très haute significativité. Voir figure 2A.
Conclusion : efficacité très élevée de la tritoqualine avec une haute significativité
La tritoqualine aussi bien à la dose de 20 ou 40 mg/kg diminue fortement le score histologique et ceci est fortement significatif. La pirfenidone est à la limite de la significativité (P 0.045)) alors que pour la tritoqualine la différence est à hautement significative (P = 0,0001 pour la dose de 20 mg/kg)- Voir la figure 2B.

La titration du collagène (méthode par le dosage de l'hydroxyproline) est exprimée en µg/mg de protéines.

### Test pirfenidone 100 mg/kg versus bléomycine pour le collagène (hydroxyproline).

- Unpaired t test(test de student)
- P value 0, 0450
- P value résumé : Le P < 0.05 Le test est-il significatif? Oui

Le test est-il hautement significatif? Non, la différence est faible (P= 0.045) à la limite de la significativité.

Moyenne ± écart type (bléomycine) : 340,0 ± 4,888, n=10
Moyenne ± écart type (Pirfenidone) : 316,5 ± 9,748, n=10
Différence entre les deux moyennes : -23,50 ± 10,90
Conclusion : efficacité de la pirfénidone à la limite de la significativité (P 045).

### Test tritoqualine 20 mg/kg versus bléomycine pour le collagène (hydroxyproline).

- Unpaired t test (test de student)
- P value < 0, 0001
- P value résumé: Le P < 0.0001 Le test est-il significatif? Oui

Le test est-il hautement significatif? Oui, la différence est très élevée (P= 0.0001) très haute significativité.
Moyenne ± écart type (bléomycine) : 340,0 ± 4,888, n=10
Moyenne ± écart type (Trito 20 mg/kg) : 280,0 ± 7,528, n=10
Différence entre les deux moyennes : -60,00 ± 8,975
Conclusion : efficacité très élevée de la tritoqualine avec une haute significativité.

La tritoqualine a une action forte sur l'inhibition de la formation du collagène qui est très supérieure à celle de la pirfénidone. La significativité est également importante (P=0, 0001)- Voir le figure 2A.

Le dosage des cytokines : La tritoqualine inhibe fortement également le TGFβ. (P= 0.001) comme la pirfenidone (P= 0.001) voir la figure 1A.

La tritoqualine pourrait être utilisée sous différentes formes, en dehors de sa forme « comprimé » sans modifier son efficacité, par exemple sous forme de gélule, sirop, gel oral, capsule ou comprimé à délitement programmé.

La tritoqualine a déjà été utilisée chez l'homme à la dose de 3 mg/kg, mais jamais dans la fibrose pulmonaire idiopathique, cependant cette dose devrait être active chez l'homme dans cette pathologie.

En effet les doses utilisées chez l'animal sont en général 10 fois supérieure aux doses utilisées chez l'homme. L'Esbriet est par exemple utilisé à la dose de 100mg/kg chez la souris alors que chez l'homme la dose utilisée est de seulement 15 mg/kg.

Ce rapport de dose est sans doute le même pour la tritoqualine. Les doses qui pourrait être efficaces seraient alors de 3 à 40 mg/kg.

## Revendications

1. Substance ayant une activité agoniste sur le récepteur H4 à l'histamine pour son utilisation dans le traitement de la fibrose pulmonaire idiopathique, **caractérisée en ce qu'**elle est constituée d'un isomère ou d'une mixture d'isomères de la 7-Amino-4, 5, 6-triethoxy-3-(5, 6, 7, 8-tetrahydro-4-methoxy-6-methyl-1,3-dioxolo[4,5-g]isoquinolin-5-yl) phthalide, ou tritoqualine .

2. Substance pour son utilisation selon la revendication 1, **caractérisée en ce qu'**elle inhibe le TGFβ.

3. Substance pour son utilisation selon la revendication 1 ou 2, **caractérisée en ce qu'**elle est conditionnée sous différentes formes galénique : gélules, comprimés, capsules, sirop, gel.

## Patentansprüche

1. Substanz, die eine Wirkung als Antagonist auf dem Rezeptor H4 gegenüber Histamin aufweist, für deren Verwendung bei der Behandlung von idiopathischer Lungenfibrose, **dadurch gekennzeichnet, dass** sie aus einem Isomer oder einem Isomer-Gemisch von 7-Amino-4,5, 6-Triethoxy-3-(5, 6, 7, 8-Tetrahydro-4-Methoxy-6-Methyl-1,3-Dioxolo[4,5-g]Isochinolin-5-yl) Phthalid, oder Tritochalin gebildet ist.

2. Substanz für deren Verwendung nach Anspruch 1, **dadurch gekennzeichnet, dass** sie TGFβ hemmt.

3. Substanz für deren Verwendung nach Anspruch 1 oder 2, **dadurch gekennzeichnet, dass** sie unter unterschiedlichen galenischen Formen konditioniert ist: Gelatinekapseln, Tabletten, Kapseln, Sirup, Gel.

## Claims

1. Substance having an agonist activity on the histamine H4 receptor for its use in the treatment of idiopathic pulmonary fibrosis, **characterised in that** it is comprised of an isomer or of a mixture of isomers of 7-Amino-4,5,6-triethoxy-3-(5,6,7,8-tetrahydro-4-methoxy-6-methyl-1,3-dioxolo[4,5-g]isoquinolin-5-yl) phthalide, or tritoqualine.

2. Substance for its use according to claim 1, **characterised in that** it inhibits TGFβ.

3. Substance for its use according to claim 1 or 2, **characterised in that** it is packaged under different dosage forms: soft capsules, tablets, capsules, syrup, gel.
